# EUROPEAN PATENT APPLICATION

(11) **EP 2 343 541 A1**
(43) Date of publication of application: **13.07.2011**
(21) Application number: 09180491.4
(22) Date of filing: 22.12.2009
(51) Int. Cl.: G01N 27/22, G01N 33/00

(54) **Sensor**

(71) Applicant: NXP B.V., 5656 AG Eindhoven (NL)
(72) Inventor: HUMBERT, Aurelie, Redhill, Surrey RH1 1DL (GB); MERZ, Matthias, Redhill, Surrey RH1 1DL (GB); PONOMAREV, Youri, Victororvitch, Redhill, Surrey RH1 1DL (GB); DAAMEN, Roel, Redhill, Surrey RH1 1DL (GB)
(74) Representative: Burton, Nick

(57) **Abstract**

Sensors fabricated using integrated circuit technology is only sensitive to a relatively small range of concentration. This can be either in the parts per million (PPM) range or percentage range. A sensor (20) is described which has a first group of electrodes (22) which are sensitive to a different range of substance concentration to a second group of electrodes (24). A sensor (20) fabricated according to the invention can be fabricated on a standard CMOS process and is sensitive to a wide range of substance concentration from a few parts per million to percentage range.

## Description

This invention relates to sensors for detecting the concentration of a substance.

It is known that sensors can be fabricated using integrated circuit technology. These sensors typically have a layer into which a substance, for example a gas or liquid to be detected can diffuse. This changes the electrical property of the layer altering the resistance, capacitance or complex impedance, which can then be detected giving an indication of the concentration of a given substance present in the atmosphere. The concentrations of the substance to be detected vary depending on the application. For some applications such as checking bacterial growth, the concentration of a gas to be detected is in the parts per million (ppm) range. For other applications where the atmosphere is modified for example for food stored in modified atmosphere packages, then the relative concentration of gases such as O2 and CO2 need to be monitored in the percentage range.

Currently available sensors are capable of detecting concentrations in specific ranges.

For example, A.Star et al, ( Adv Materials Vol. 16 N22, p2049, 2004 ) describe a FET based CO2 sensor having a fast response to CO2 gas at low concentration and a wide dynamic range for C2 concentration from 500ppm to 10% in air.

Jaime Herran et al describe a solid state gas sensor for fast carbon dioxide detection, (Sensors and Actuators B, Vol. 129, pp 705-709, 2008) having a linear behaviour between 500 and 2000ppm of carbon dioxide but saturation behaviour for concentrations above 5000ppm.

Wen Wang et al (Smart material structures Vol. 16, pp 1382-1389, 2007) describe a novel wireless, passive CO₂ sensor incorporating a surface acoustic wave reflective delay line with good linearity and repeatability for a CO₂ concentration of 0-450 ppm.

H.E.Endres and al ( Sensors and Actuators B Vol. 57, pp 83-87, 1999) describe a capacitive CO₂ sensor system with the suppression of the humidity interference with a CO₂ measurement range having a high resolution between 100 and 3000ppm and a lower resolution 100-10000ppm CO₂.

K.Obata and Al (Sensors and Actuators B 76 (2001) 639-643) describe a potentiometric sensor for detection of CO₂ at room temperature. The EMF of the device is linear to the logarithm of CO₂ concentration in the range 300-3000ppm.

It is therefore desirable to provide an improved sensor for detecting a wider range of concentrations of a substance.

### SUMMARY OF INVENTION

Various aspects of the invention of the invention are defined in the accompanying claims.

According to a first aspect of the invention, there is provided a sensor for detecting a substance, the sensor comprising a first and second groups of electrodes arranged on a substrate, wherein the first group of electrodes is configured to detect substance in a first range of concentrations, and wherein the second group of electrodes is configured to detect substance in a second, different range of concentrations.

Having multiple groups of electrodes with different configurations enables the sensor to detect a wide range of concentrations (e.g. from a few ppm to percentage levels) without saturating.

In some embodiments of the invention, the first group of electrodes has a first inter-electrode spacing, and the second group of electrodes has a second, different inter-electrode spacing. Varying the electrode spacing alters the sensitivity of a group of electrodes to substance concentrations. A narrower spacing between a particular group of electrodes results in that region of the sensor being sensitive to detect higher concentrations of substance. This is because a higher concentration of substance is required to cause a detectable change in the electrical properties of the sensor in that region by, for example, changes in capacitance or resistance.

Further embodiments of the invention have a substance sensitive layer adjacent to the electrodes. By having a depth or thickness of the substance sensitive layer adjacent to the first group of electrodes, which is different to the depth or thickness of the substance sensitive layer adjacent to the second group of electrodes, the sensor is able to detect a wide range of substance concentrations. A group of electrodes in a region of the sensor having a deeper or thicker substance sensitive layer results in that regions being sensitive to higher concentrations of substance.

Embodiments of the invention will now be described in detail illustrated by way of example only by the accompanying drawings in which:
Figure 1 shows a plan view and cross sectional view of a known sensor.
Figures 2a, 2b, 2c, 2d illustrate cross section of a sensor in accordance with one embodiment of the invention.
Figure 3 shows a graph illustrating an example of the variation in capacitance with electrode spacing in accordance with an embodiment of the invention.
Figures 4a, 4b, 4c, 4d illustrate a cross section of a sensor and the fabrication steps in accordance with a further embodiment of the invention.
Figure 5 shows a graph illustrating an example of the variation of capacitance with thickness of substance sensitive layer in accordance with a further embodiment of the invention.
Figure 6 shows the cross section of a sensor in accordance with an embodiment of the invention.

### DESCRIPTION

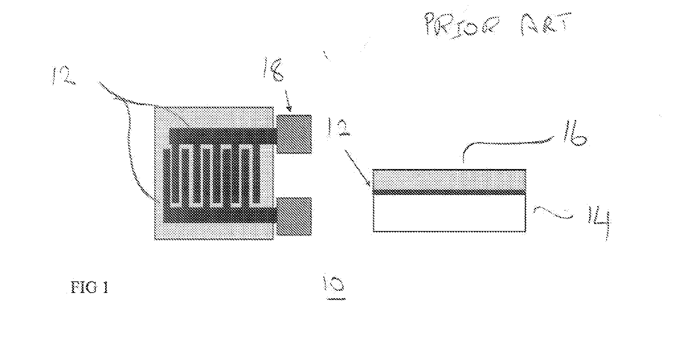

Figure 1 shows an example of a known capacitive sensor 10. A structure composed of two interdigitated electrodes (IDE) 12 is formed on a substrate 14. A dielectric layer 16 is formed above the interdigitated electrodes 12. The interdigitated electrodes 12 are each connected to a bonding pad 18. When a substance such as water vapour diffuses into the dielectric layer it causes the dielectric constant to change and this change causes a corresponding change of capacitance which can be detected.

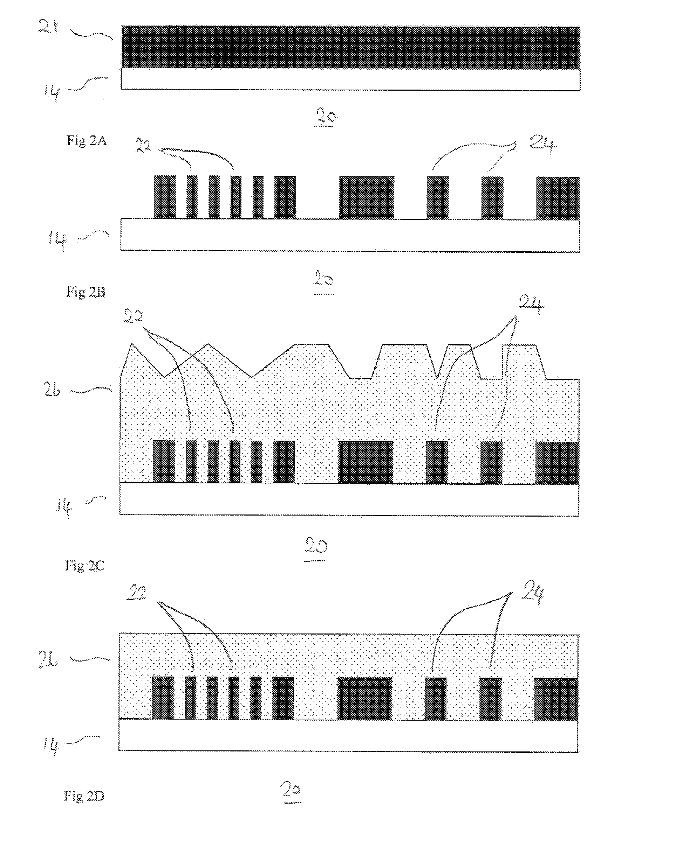

Figures 2a to 2d illustrate an example sensor 20 according to an embodiment of the invention. A metal layer 21 can be deposited on a substrate 14 as shown in figure 2a. The substrate 14 can be silicon or a processed integrated circuit. Metal layer 21 is patterned using, for example, standard CMOS processing steps to form a first group of electrodes 22 and a second group of electrodes 24 as shown in figure 2b.

A layer of substance sensitive material 26 is deposited on the metal layer 21 surrounding the first group of electrodes 22 and a second group of electrodes 24 as shown in figure 2c.

The actual material used in the substance sensitive layer 26 can be chosen according to the substance to be detected. Table 1 indicates a number of substances that can be detected in accordance with embodiments of this invention, and a number of alternative types of material that can be used in the substance sensitive layer 26 to detect those substances. The substances to be detected can be water vapour for humidity measurement, CO2, O2, Ethylene and NH₃.

**Table 1**

| Substance | Capacitive type |
|---|---|
| Humidity | Al₂O₃, polyimide, TiO₂, SiO₂, SiC, polyesters, PMMA (Polymethyl methacrylate), B C B (Benzocyclobutene), polysulfates, cellulose acetate butyrate, porous silicon |
| CO₂ | Fluoropolymer, CuO mixed with BaSnO₃, SrTiO₃, CaTiO₃, ZnO or BaTiO₃, N-H containing polymers |
| O₂ | Zirconium oxide, Irridium oxides |
| Ethylene | SnO₂ based film |
| NH₃ | Porous SiC, TiO₂ |

Further examples of suitable sensitive materials can be found in, for example, Table 2 of "Materials Used as Chemical Sensor Elements" in "Chemical Sensor Technology", Vol.1, Ed. Tetsuro Seiyama, Elsevier 1988.

The substance sensitive layer 26 can be planarised as illustrated in figure 2d. The first group of electrodes 22 can have a different spacing to the second group of electrodes 24. The substance sensitive layer 26 can follow the topography of the electrodes 22, 24 dependent on the spacing.

For all embodiments described herein, the thickness of the substance sensitive layer is the dimension between a surface of the substance sensitive layer 26 which is in first contact with the substance, and the surface of the substance sensitive layer 26 which is opposite to the surface of the substance sensitive layer that is in first contact with the substance.

Depending on the spacing, the topography of the sensitive layer of the sensor area differs, and may or may not follow the metal line pattern. The variation in electrode spacing and the topography of substance sensitive layer 26 can result in different increases in capacitance value when a substance diffuses into the substance sensitive layer 26. The spacing between electrodes can vary between 50 nanometres to 4 microns.

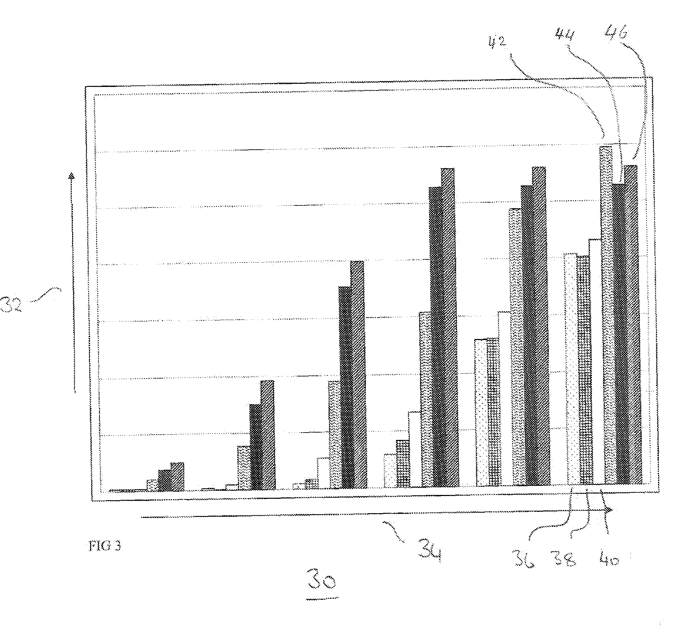

Figure 3 shows a graph 30 illustrating an example of the percentage increase in capacitance (on the y-axis 32) versus increasing diffusion depth of a substance into the substance sensitive layer 26 (on the x-axis 34). The substance sensitive layer 26 in this graph is fixed to a thickness of 1 micron. Six examples of electrode spacing are used, which are 0.25 microns 36, 0.5 microns 38, 1 micron 40, 2 microns 42, 3 microns 44 and 4 microns 46. The depth that the substance diffuses into the substance sensitive layer correlates to the concentration of the substance in the atmosphere surrounding sensor 20.

For a substance diffusing to a depth of 100 nanometres into the substance sensitive layer 26, the resulting capacitance increase varies between 0.15 percent for 0.25 micron electrode spacing, to 2.44 percent for 4 micron electrode spacing. Where the substance has diffused to a depth of 400 nanometres into the substance sensitive layer 26, the increase in capacitance varies from 0.17 percent for a 0.25 micron electrode spacing up to 9.55 percent for 4 micron electrode spacing. For 800 nanometres diffusion into the substance sensitive layer 26 the resulting increase in capacitance is 0.43 percent for 0.25 micron electrode spacing up to 19.97 percent for 4 micron electrode spacing. For 1000 nanometres diffusion into the substance sensitive layer 26 the capacitance increases by 2.95 percent for 0.25 micron electrode spacing and 28.07 percent for 4 micron electrode spacing. For 1500 nanometres diffusion into substance sensitive layer 26, the capacitance increases by 12.87 percent for 0.25 micron electrode spacing up to 28.07 percent for 4 micron electrode spacing. When the substance is diffused to 2000 nanometres into the substance sensitive layer 26 the 0.25 micron spaced electrodes increase by 20.34 percent and the 4 micron spaced electrodes have increased by 28.07 percent.

Hence, by having an electrode spacing of 4 microns 46, the substance sensor 20 is sensitive to small changes in concentration but then saturates at large substance concentrations. Conversely for electrode spacing of 0.25 microns 36, the substance electrode will be insensitive to small concentrations of substance in the ppm range but will then be sensitive to large concentrations of substance in the percentage range. This illustrates that having a first group of electrodes 22 having a small spacing, for example 0.25 microns, can be used for high percentage substance concentration measurement and having a second group of electrodes 24 having a larger spacing, for example 4 microns, the substance sensor 20 will be more sensitive to ppm changes in substance concentration. Therefore, by having groups of electrodes with different spacing between the electrodes, it is possible to have a single substance sensor that is sensitive to substance concentrations from the ppm range to the percentage range.

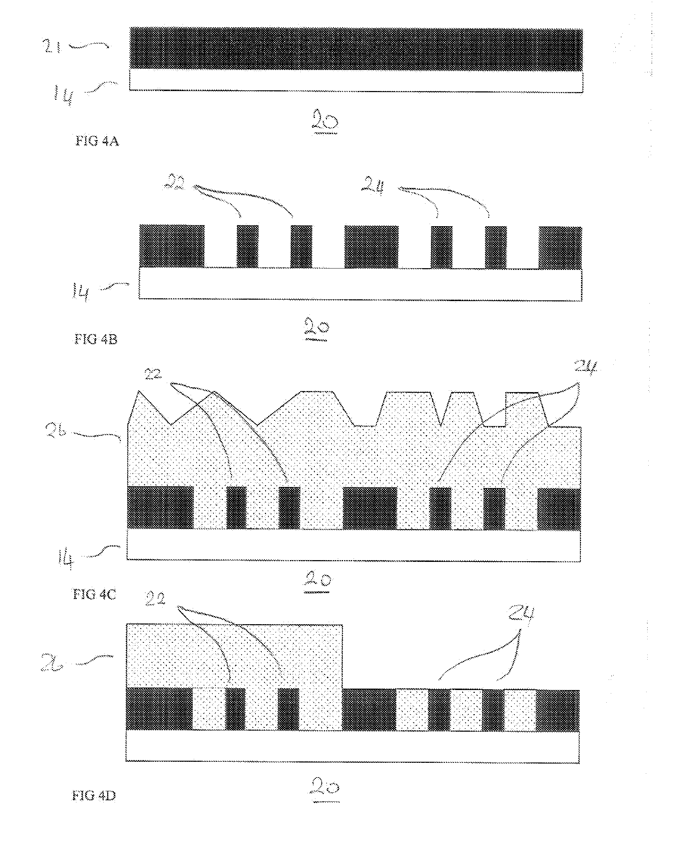

Figures 4a to 4d illustrate the steps to manufacture a sensor 20 in accordance with a further embodiment of the invention. A metal layer 21 is deposited on a substrate 14 shown in figure 4a. The substrate can be silicon or an integrated circuit or device. Metal layer 21 is patterned to form first group of electrodes 22 and a second group of electrodes 24 using, for example, standard CMOS processing steps. The first group of electrodes 22 can have the same spacing as the second group of electrodes 24. Substance sensitive layer 26 is deposited surrounding the first group of electrodes 22 and a second group of electrodes 24 as shown in figure 4c. The substance sensitive layer 26 is partially etched to form a layer 26 having different thickness above and around the first group of electrodes 22 compared to the second group of electrodes 24 as shown in figure 4d. The thickness of the substance sensitive layer 26 can vary between 20 nanometres and 5 microns.

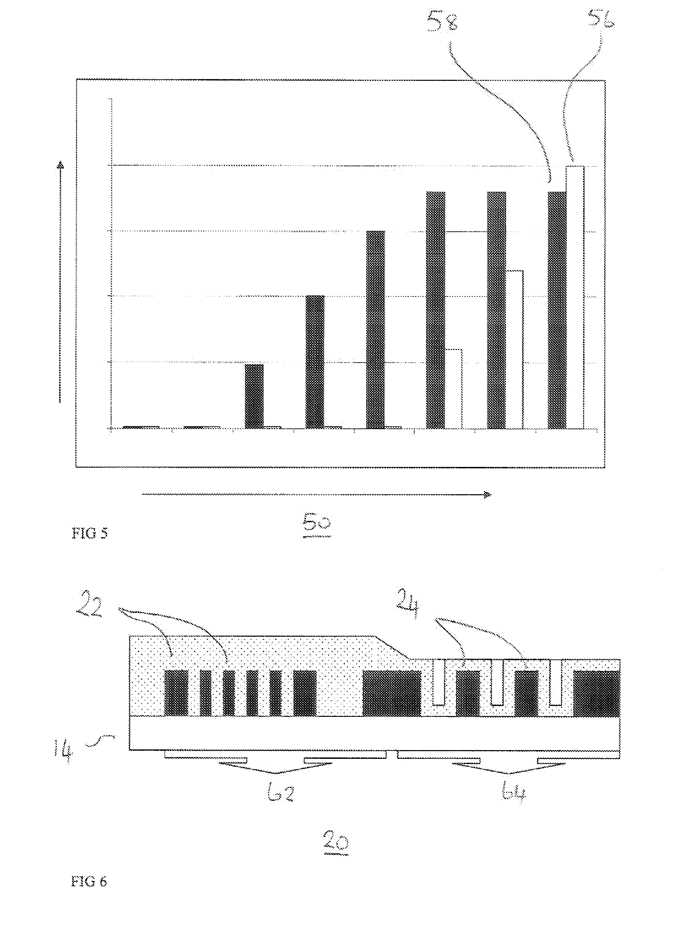

Figure 5 shows a graph 50 showing the relative increase in capacitance on y-axis 52 versus increasing diffusion depth of a substance into the substance sensitive layer 26 on x-axis 54. This illustrates the effect of varying the thickness of the substance sensitive layer 26 on the sensitivity of sensor 20. Two structures have the same electrode design but the sensitive layer 26 thickness differs. In this example, the sensitive layer is polyimide with the dielectric constant of 2.5 and for this example the substance is H₂0. On absorption of moisture, the polyimide dielectric constant changes to 3.1. In first structure 56, the thickness of the polyimide is 1.9 microns whereas in second structure 58, it is 0.9 microns. Both structures are exposed to six different concentrations giving six different diffusion thicknesses (100 nanometres, 400 nanometres, 600 nanometres, 800 nanometres, 1200 nanometres, 1500 nanometres, 1900 nanometres) structure 56 shows no detectable increase in capacitance until the diffusion depth in the sensitive layer is 1200 nanometres and hence cannot detect small concentrations. Structure 58, which has a thinner sensitive layer, starts to have a measurable capacitance change when the diffusion in the sensitive layer reaches 400 nanometres, therefore this can be used to detect lower concentrations of substance. However, second structure 58 saturates once the diffusion depth reaches the thickness of the substance sensitive layer 26 of 900 nanometres and is therefore not sensitive to concentrations corresponding to diffusion depths greater than 900 nanometres.

By having a thickness of the substance sensitive layer 26 surrounding a first group of electrodes 22 which is different compared to the thickness of the substance sensitive layer 16 surrounding the second group of electrodes 24, a sensor 20 can detect changes in substance concentration in ppm and percentage ranges.

Figure 6 shows further example of sensor 20 in accordance with another embodiment of the invention. A first group of electrodes 22 and a second group of electrodes 24 is formed above the substrate 14. The substrate 14 can be silicon or a processed integrated circuit. The first group of electrodes 22 are spaced more closely together than the second group of electrodes 24. A substance sensitive layer 26 is deposited above and around a first group of electrodes 22 and a second group of electrodes 24. The substance sensitive layer 26 follows the metal line pattern in the second group of electrodes 24. The substance sensitive layer 26 does not follow the topography of the metal line pattern over the first group of electrodes 22. Substance sensitive layer 26 is thicker in region 62 of the first group of electrodes 22 compared to region 64 of the second group of electrodes.

The combination of varying electrode spacing and thickness of substance sensitive layer 26 results in region 62 ( containing the first group of electrodes 22 ) being sensitive to higher levels of substance concentration than region 64 (containing the second group of electrodes 24).

By having groups of electrodes having spacing varying from 50 nanometres to 4 microns, and a thickness of substance sensitive layer varying from 20 nanometres to 5 microns, a sensor can be fabricated which is sensitive to substance concentrations from a few ppm to percentage range without saturating.

Embodiments described herein are by way of example only and it will be appreciated that further embodiments are possible. For example, more than two groups of electrodes can be integrated onto a single device to detect plurality of ranges of concentrations of substance. A sensor does not have to be sensitive to a contiguous range of concentration of substance. For example, a sensor can be fabricated which is sensitive to concentrations in the range of 1 to 100ppm and 5 to 10 percent. Because the sensors are all integrated on the same chip area, the output of any sensor can be used as input data for another sensor on the chip. For example, CO₂ sensing materials can be cross sensitive to water. The sensor can include a separate humidity sensor, and electronics can be used to compensate for the moisture interaction. Since embodiments of the invention can be fabricated using standard CMOS processing, the invention can be used to form CMOS RFID sensors. The substrate material does not have to be silicon; any material on which groups of electrodes (22, 24) and a substance sensitive layer 26 can be deposited, may be suitable as a substrate.

Accordingly, there has been described a sensor (20) having a first group of electrodes (22) which are sensitive to a different range of substance concentration to a second group of electrodes (24). A sensor (20) fabricated according to the invention can be fabricated on a standard CMOS process and can be sensitive to a wide range of substance concentration from a few parts per million to percentage to a percentage range.

## Claims

1. A sensor for detecting a substance, the sensor comprising:
first and second groups of electrodes arranged on a substrate,
wherein the first group of electrodes is configured to detect the substance in a first range of concentrations, and wherein the second group of electrodes is configured to detect the substance in a second, different range of concentrations.

2. The sensor of claim 1, wherein the first group of electrodes has a first inter-electrode spacing, and wherein the second group of electrodes has a second, different inter-electrode spacing.

3. The sensor of claim 2, wherein the first inter-electrode spacing is narrower than the second inter-electrode spacing.

4. The sensor of claim 3, wherein the first inter-electrode spacing is in the range of 0.05 microns to 2 microns, and the second inter-electrode spacing is in the range of 0.05 microns to 4 microns.

5. The sensor of any preceding claim comprising at least one further group of electrodes configured to detect substance in a further range of concentrations.

6. The sensor of any preceding claim, comprising a substance-sensitive material adjacent to the electrodes.

7. The sensor of claim 6, wherein the minimum thickness of the substance-sensitive material adjacent to the first group of electrodes is different to the minimum thickness of the substance-sensitive material adjacent to the second group of electrodes.

8. The sensor according to claim 7, wherein the minimum thickness of the substance-sensitive material adjacent to the first group of electrodes is at least 0.1 micron and the minimum thickness of the substance-sensitive material adjacent to the second group of electrodes is greater than the minimum thickness of the substance-sensitive material adjacent to the first group of electrodes.

9. The sensor according to any preceding claim wherein the first range of substance concentrations and the second range of substance concentrations are non-contiguous.

10. The sensor of claim 9, wherein the first range of substance concentrations is 1 to 1 000 ppm and the second range of substance concentrations is 1 % to 100 %.

11. The sensor of any of claims 1 to 8 wherein the first range of substance concentrations is 1 to 10000 ppm and the second range of substance concentrations is 1 % to 100 %.

12. The sensor of any preceding claim wherein the substance to be detected is at least one of H₂O, CO₂, O₂, C₂H₄ and NH₃.

13. An Radio Frequency Identification (RFID) tag comprising the sensor of any preceding claim.

14. An array of CMOS devices comprising the sensor of any of claims 1 to 12.

15. An integrated circuit comprising the sensor of any of claims 1 to 12.
